(19) Europäisches Patentamt
European Patent Office
Office européen des brevets

(11) Publication number: **0 153 533**
**B1**

(12) # EUROPEAN PATENT SPECIFICATION

(45) Date of publication of patent specification: **16.03.88**

(21) Application number: **84308722.2**

(22) Date of filing: **14.12.84**

(51) Int. Cl.⁴: **C 07 C 125/063,**
C 07 C 125/07, C 07 C 127/19,
C 07 C 155/02, C 07 C 157/09,
C 07 C 161/04, G 01 N 33/53,
C 07 J 41/00

(54) **Rigid coupling compounds.**

(30) Priority: **06.01.84 US 568482**

(43) Date of publication of application:
**04.09.85 Bulletin 85/36**

(45) Publication of the grant of the patent:
**16.03.88 Bulletin 88/11**

(84) Designated Contracting States:
**BE DE FR GB IT NL SE**

(56) References cited:
**EP-A-0 078 952**
**EP-A-0 113 538**
**DE-A-2 415 435**
**DE-A-2 526 984**
**DE-A-2 722 038**
**DE-A-2 837 087**
**DE-A-3 110 573**
**DE-B-2 743 446**
**DE-C- 899 192**
**FR-A-2 262 670**
**GB-A-1 122 445**
**GB-A-1 179 231**
**GB-A-1 315 871**

(73) Proprietor: **Becton, Dickinson and Company**
**Mack Centre Drive P.O. Box 2224**
**Paramus New Jersey 07652-1149 (US)**

(72) Inventor: **Allen, Stephen D.**
**P.O. Box 2835, 2199 Monarch**
**Park City, UT (US)**
Inventor: **Thompson, Michael**
**6101 Alexandria Drive**
**Evansville, IN 47714 (US)**

(74) Representative: **Ruffles, Graham Keith et al**
**MARKS & CLERK 57-60 Lincoln's Inn Fields**
**London WC2A 3LS (GB)**

(56) References cited:
**GB-A-2 069 997**
**US-A-3 036 112**
**US-A-3 081 310**
**US-A-3 721 700**
**US-A-3 783 151**
**US-A-3 979 426**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 153 533 B1

**Description**

This invention relates to the coupling of one organic material to another, intermediates useful in producing coupled products, and uses for such coupled products.

Coupling agents or spacer compounds are generally bifunctional compounds which are employed for coupling one organic material to another. For example, in an assay, such as an immunoassay, one of the components of the assay is a tracer which is comprised of a ligand; for example, an antigen, coupled to a suitable marker; for example, a chromogen, such as a fluorescent dye. In producing such a tracer, in many cases, the antigen is coupled to the fluorescent dye by use of a bifunctional coupling agent or spacer.

Similarly, in an immunoassay employing a solid support, there is a need to couple a material used in the assay, such as an antibody, to a solid support, such as a polymer, and in some cases, this is accomplished by use of a coupling agent.

Examples of known coupling agents are to be found for instance in the following patent specifications: European 78 952; German 2 722 038, 2 837 087 and 2 743 446; and French 2 262 670.

There is a need in the art for improved means of coupling one material to another through a coupling agent or spacer compound.

In accordance with one aspect of the present invention, there is provided intermediates for coupling one material to another.

In accordance with another aspect of the present invention, there is provided coupled compounds or materials.

In accordance with a further aspect of the invention, there is provided a process for preparing such coupling agents, intermediates and coupled compounds.

In accordance with yet another aspect of the invention, there is provided a process for using coupling agents, intermediates and coupled products.

More particularly, in accordance with one aspect of the present invention, there is provided an intermediate useful in preparing coupled compounds, which has the following structural formula:

$$R—CO—NH—Y—A \qquad (I)$$

wherein

Y is a divalent aromatic hydrocarbon radical;

R is an organic radical having at least one active hydrogen substituent group (in particular, an amine, thiol or hydroxyl substituent group); and

A is selected from the group consisting of $—NO_2$, $—NH_2$, —COOH, —CO—Cl, —CO—OR'', —N=C=O, —N=C=S, —SH, —OH, —S—CO—R'', and —O—CO—R'', where R'' is alkyl.

In these coupling compounds of formula (I), the radical R is a detectable marker, an antibody, a hapten or an antigen.

In accordance with another aspect of the present invention, there is provided coupled compounds having the following structural formula:

$$R—CO—NH—Y—Z \qquad (II)$$

wherein

Z is selected from the group consisting of

—NH—CO—B, —NH—CO—O—B,
—NH—CO—S—B, —NH—CO—NH—B,
—NH—CS—NH—B, —CO—NH—B,
—S—CO—NH—B, —O—CO—NH—B,

where

B is an organic radical;

R is an organic radical having at least one active hydrogen substituent group wherein R is coupled through the active substituent group (preferably an amine, thiol, or hydroxyl substituent group); and

Y is a divalent aromatic hydrocarbon radical.

In the compounds (II), one of R and B comprises a detectable marker and the other comprises an antigen, hapten or antibody.

In accordance with a particularly preferred embodiment of the invention, in the hereinabove described structural formulas, Y is a divalent benzene radical, although it is also to be understood that Y could be a divalent naphthalene or a divalent diphenyl radical.

The intermediates (I) may be prepared from compounds represented by structural formula (III):

$$O=C=N—Y—R' \qquad (III)$$

where R' is $—NO_2$, —COOR'', —S—CO—R'' or —O—CO—R'' wherein R and Y are as hereinabove defined.

R'' is generally in either the para- or meta-position, preferably para, with R' most preferably being $—NO_2$.

In the case where in Compound III, R' is $NO_2$, Compound III is initially coupled to an organic compound having an active hydrogen substituent group which is reactive with an isocyanate group to produce a Compound I wherein A is $—NO_2$. Subsequently, the $—NO_2$ group may be selectively reduced to an amino group (use of sulfurated sodium borohydride) to provide a reactive group for coupling to another organic compound. Alternatively, the amino group may be converted to an isocyanate (reaction with phosgene) or isothiocyanate group (reaction with thiophosgene); either of which are reactive for coupling intermediate I to an organic compound.

In the case where, in Compound III, R' is —COOR'',

$$—S—\overset{\overset{O}{\|}}{C}—R'', \text{ or } —O—\overset{\overset{O}{\|}}{C}—R'',$$

after coupling Compound III to an organic compound through the isocyanate substituent group,

substituent group R′ may be hydrolyzed to provide a substituent group A which is either —COOH, —SH or —OH, respectively, each of which is reactive for coupling intermediate I to an organic compound.

Thus, as should be apparent, Compound III is selected so as to include a substituent group R′ which is not reactive with the active hydrogen substituent group of the organic compound to which Compound III is to be coupled through its isocyanate group. Subsequently, R′ is converted to a substituent group A which is reactive with an active hydrogen substituent group of the organic compound to which intermediate I is to be coupled so as to produce coupled compound II.

The compound represented by structural formula I may be employed for producing coupled compounds, as represented by structural formula II. In using the compound represented by structural formula I, the substituent group represented by A is one which is capable of reacting with an active hydrogen substituent group on the compound to which the compound represented by structural formula I is to be coupled. Thus, for example, when A is an amino group, compound I may be coupled to an organic compound having a carboxyl substituent group by procedures generally known in the art. Similarly, when A is carboxyl, compound I may be coupled to an organic compound which has either an amino substituent group or an isocyanate substituent group by procedures known in the art. When A is isocyanate, compound I may be coupled to an organic compound which has an active hydrogen substituent group which is either mercapto (thiol), hydroxy, carboxyl or amino. When A is thiol or hydroxy, compound I may be coupled to an organic compound which has an active hydrogen substituent group which is isocyanate. When A is isothiocyanate, compound I may be coupled to an organic compound which has an amino substituent group. The procedures for accomplishing such coupling through such functional groups are generally known in the art.

The coupling agents III may be employed for coupling a wide variety of organic materials to each other. Thus, for example, the coupling compounds III may be employed for producing tracers which are to be used in an assay wherein such tracer is comprised of a detectable marker; e.g., a radioactive marker, chromogen, enzyme, etc., coupled to a ligand (the term "ligand" as used herein refers to a hapten, antigen or antibody). In such an embodiment, one of the marker or ligand includes a substituent group which is capable of reacting with an isocyanate group, and the substituent group R′ of coupling agent III is nonreactive with the substituent group on the ligand or marker. After the coupling agent III is coupled to the ligand or marker, there is produced an intermediate compound I wherein the substituent group A is nonreactive with the active hydrogen substituent group which is on the ligand or marker which has been initially coupled to produce intermediate I. The intermediate compound I is then coupled to the other of the marker or ligand, and if the substituent group A of intermediate I is not reactive with the active hydrogen substituent group on the other of the ligand or marker, then the substituent group A is selectively converted to a substituent group, as defined, which is reactive with the active hydrogen substituent group on the other of the ligand or marker. The intermediate I is then coupled to the other of the ligand or marker to produce a coupled compound II.

Thus, in producing a tracer for use in an assay, in coupled compounds II, one of R and B is a marker, such as a chromogen, an organic compound including a radioactive substituent group or an enzyme, and the other of R and B is a ligand. Thus, for example, in producing a fluorescent tracer, one of R and B is derived from a fluorescent dye having a substituent group which can be coupled to either isocyanate, or one of the reactive functional groups represented by A in structural formula I, and the other or R and B is derived from a ligand having a substituent group which can be coupled to the other of the isocyanate or reactive substituent group represented by A in structural formula I. In producing such a tracer, either the ligand or the fluorescent dye can be initially coupled to the isocyanate functional group of coupling agent III.

Applicant has found that the use of a coupling agent represented by structural formula III is particularly advantageous for producing fluorescent tracers, in that the coupling agent is rigid, whereby the fluorescent marker does not "fold" back onto the ligand, thereby minimizing the possibility of quenching of the fluorescent compound by the ligand.

As representative examples of suitable chromogens, there may be mentioned: acridine dyes, azure dyes, quinone dyes, Nile blue, Cresyl Violet, fluoresceins, rhodamines, coumarines, amino naphthalene derivatives (dansyl compounds), carbocyanines, indoles, lanthanide chelates, etc.

Thus, for example, a $T_4$ tracer may be prepared by initially coupling p-nitrophenylisocyanate to thyroxine ($T_4$) wherein the carboxyl moiety of the $T_4$ is appropriately blocked. The nitro group is reduced to amine (Compound I, R is blocked $T_4$ radical, Y is benzene and A is amine), and the amino portion of compound I may be coupled to a fluorescent dye including, for example, an isothiocyanate group; in particular fluorescein isothiocyanate.

Alternatively, the amino group of Compound I may be converted to isothiocyanate by reaction with thiophosgene (in Compound I A is isothiocyanate), followed by reaction of Compound I with a fluorescent dye including an amino group; in particular fluorescein amine.

Similarly, a digoxin tracer may be produced by coupling digoxin to p-nitrophenylisocyanate, followed by reduction of nitro to amine, and reaction with a fluorescent dye, such as fluorescin isothiocyanate.

Although the present invention has particular

use for producing fluorescent tracers, as hereinabove noted, the present invention may also be employed for producing other tracers, such as radioactive tracers, enzyme tracers, etc. As representative examples of suitable radioactive markers for producing radioactive tracers, there may be mentioned: hydroxyphenyl substituted amines or amino acids, wherein the phenyl group includes one or more radioactive substituent groups, such as radioiodinated tyrosine or tyramine; imidazole substituted amino acids or amines wherein the imidazole group is substituted with one or more radioactive substituent groups, and the like.

As representative examples of suitable enzyme markers, there may be mentioned: peroxidases, β-galacosidase, acetylcholine esterase, glucoamilase, maleic acid dehydrogenase, glucose-6-phosphoric acid dehydrogenase, glutaroxidase, acid phosphatase, etc.

As known in the art, the solid support may be polymer, provided that the polymer includes a substituent group capable of reacting with either the isocyanate functionality or one of the reactive substituents represented by A of intermediate I; e.g., polyacrylamide, poly (aminostyrene), etc.

The tracers and supported ligands prepared in accordance with the present invention may be employed in an assay for a wide variety of analytes (the term "analytes" refers to a hapten, antigen or antibody), of a type generally known in the art. Thus, for example, the present invention is applicable to assays for drugs, including therapeutic drugs and so-called "drugs of abuse"; steroids, vitamins, sugars, amino acids, polypeptides, proteins, various hormones, antibiotics, viruses, etc.

The tracers and supported ligands prepared in accordance with the present invention may be employed in an immunoassay (the term "immunoassay" is used in a generic sense and includes assays which use naturally occurring binders instead of an antigen or antibody, and which are sometimes referred to as competitive protein binding assays), and as known in the art, one of the components of the assay is a binder. In the case where the analyte is a hapten or antigen, the binder may be an antibody or naturally occurring substance which has one or more binding sites specific for the analyte, and in a case where the analyte is an antibody, the binder may be an antigen to the antibody or an antibody elicited in response to the analyte. The selection of a suitable binder is deemed to be within the scope of those skilled in the art, and no further details in this respect are deemed necessary for a complete understanding of the invention.

In the assay, the ligand portion of the tracer used in the assay is determined by the type of assay to be employed. Thus, for example, if the assay is for an analyte which is either an antigen or hapten, the ligand portion of the tracer is either the antigen or hapten to be assayed or appropriate analog thereof (the term "appropriate analog" means an analog of the analyte which is bound by the binder used in the assay). Alternatively, the ligand portion of the tracer may be a binder for the hapten or antigen to be assayed, in which case the assay is designed so that the analyte inhibits binding of the tracer to binding sites specific for the tracer.

In the case where the analyte is an antibody, the ligand portion of the tracer may be the antibody or appropriate analog thereof, in which case both the antibody and the tracer would compete for a limited number of binding sites specific for both the antibody analyte and the tracer. Alternatively, the ligand portion of the tracer may be an antigen to the antibody analyte or antibody elicited in response to the antibody analyte, in which case, the antibody analyte inhibits binding of the tracer to binding sites specific for the tracer.

In some cases, where the analyte is to be determined by a so-called "sandwich type" of assay, the ligand portion of the tracer has binding sites specific to the analyte, which analyte has multiple determinant sites.

The selection of a suitable ligand for use as the ligand portion of the tracer is deemed to be within the scope of those skilled in the art from the teachings herein and, accordingly, no further details in this respect are deemed necessary for a complete understanding of the present invention.

The coupling agents of the present invention may also be employed for coupling a therapeutic agent to an antibody, and in particular a monoclonal antibody. In such a case, in coupled compound II, R is derived from a therapeutic agent, and B is derived from an antibody.

Thus, as should be apparent from the hereinabove description, the coupled compound II may be produced from a wide variety of organic compounds, with the organic radical represented by R in compound II being derived from one organic compound, and the organic radical represented by B in coupled compound II being derived from another organic compound, provided that the respective organic compounds include appropriate active hydrogen substituent groups for reacting with the appropriate substituent group in coupling agent III (isocyanate) or the appropriate reactive substituent group represented by A in structural formula I. Thus, in the coupled compound II, R may be an organic radical derived from organic compounds which include an active hydrogen group capable of reacting with an isocyanate group, which organic compound may be a marker (radioactive substituted organic compound, a chromogen, preferably a fluorescent dye, an enzyme), or a ligand, and in particular, a nonprotein antigen or a nonprotein hapten, a solid support or a therapeutic agent (in particular, a drug). Similarly, B may be an organic radical derived from an organic compound which includes an active hydrogen substituent group capable of reacting with one of the reactive substituent groups represented by A in intermediate I. Thus, B may be an organic radical derived from an organic compound which may be a protein, antibody, a hapten, an antigen, a

chromogen (a dye, preferably a fluorescent dye), an enzyme, an organic compound having a radioactive substituent group, a polymer, etc.

The present invention will be further described with respect to the following examples; however, the scope of the invention is not to be limited thereby:

Example 1

2.1 millimoles of trimethylsilylchloride (TMS—Cl) is added to 1 millimole of $T_4$ and redistilled pyridine. The reaction requires about two hours and is nearly quantitative. After the reaction, 1.1 moles of p-nitrophenylisocyanate is added to the reacted mixture by injection and the mixture is stirred using a magnetic stir bar for about 48 hours, with small samples being withdrawn about every four hours or so for analysis by TLC (thin layer chromatography). After 48 hours, the product is washed extensively with methanol for removal of unreacted isocyanate, followed by separation of the products by TLC or high pressure liquid chromatography (HPLC) or by column chromatography. The resulting product is para-nitrophenylisocyanato-$T_4$ wherein the carboxyl functional group of the $T_4$ is blocked with trimethyl silane.

The above reactions are accomplished at room temperature and pressure (STP).

The nitro group is reduced to amine by addition of 0.55 millimoles (excess over the concentration of $T_4$ used) of sulfurated sodium borohydride to 0.5 millimoles of the product, at room temperature and pressure. The reaction is accomplished by mixing for about 3 to 4 hours, and monitoring conversion of nitro to amine by IR. The resulting product is para-aminophenyl isocyanato-$T_4$.

To the above product, there is added a molar equivalent of isothiocyanate-fluorescein, followed by stirring for about 48 to 72 hours, and monitoring by TLC or HPLC. The TMS blocking agent is removed under aqueous conditions after coupling of the fluorescein dye to the $T_4$ coupled intermediate.

The resulting product is a fluorescent tracer wherein fluorescin isothiocyanate is coupled to $T_4$ through a rigid coupling agent, and such tracer may be employed in an assay for $T_4$.

Example 2

Preparation of digoxin fluorescent tracer

To a 100 ml round bottom flask, add 1 millimole of digoxin and stir bar. Affix a rubber septum to opening and slowly purge the round bottom with nitrogen for 5 minutes, using a vent needle. Using a 50 ml glass syringe with a long 18-gauge stainless steel needle, withdraw 30 mls of freshly distilled pyridine and inject through the rubber septum into the round bottom, slowly at first and add stirring after 15 to 20 mls have been injected. The remaining 10 to 15 mls should be used to wash down the sides of the round bottom to get the digoxin into solution. Again, using the vent needle, purge the vessel with nitrogen for approximately 5 minutes. Continue mixing (by stirring) until all the digoxin has dissolved and is completely in solution.

In a separate vessel, preferably a closed round bottom, take 1.1 millimoles of (previously dried) para-nitro phenylisocyanate, and add by injection, 15 mls of pyridine and mix-purge the closed vessel with $N_2$, as described, and when all material is in solution, withdraw by syringe and add slowly with continuous mixing, to the 100 ml round bottom containing the digoxin.

*Note:* 1) All work should be done in a fume hood.
2) The p-nitrophenylisocyanate should be checked by IR, prior to any use. Age and exposure can greatly affect the isocyanate, and lots should be assayed for the presence of isocyanate.

Samples for TLC should be withdrawn by syringe and monitoring by TLC should begin 2 hours after addition of the p-nitrophenylisocyanate. For the first day, every 2 to 4 hours, spot checks should be done, and when left overnight, TLC should be done first thing in the morning.

The reaction takes approximately 72 hours to complete. (Although after 48 hours, the main product is substantially formed — 75% completed). If left longer, increases in the desired product will be noticed, but it is at the cost of additional side product in relative or greater proportion. To assure the best possible results, purification (and isolation) of the preferred product should be done here.

Purification of this product is by preparative TLC, and can be accomplished within one day, but care must be taken to *not overload the plates*, or the bonds will run together. Upon extraction from the silica gel, using methanol: chloroform, the solution should be filtered through a 0.22 μ filter into a round bottom for rotary evaporation. The filtering is important to remove traces of the silica gel which could break down the isocyanate (bond) formation to the digoxin. (Centrifugation of the silica gel, methanol: chloroform, prior to filtering, aids in removal of a majority of the particulates and speeds the process of filtering).

At this point, the most rapid test of assuring the product, and its relative concentration, is by RIA for digoxin. After rotary evaporation of the filtered product (4X), and taking the product up in small volume of methanol, dilutions can easily be prepared and tested for immunoreactivity of the substituted digoxin. Once this is established, and relative purity (by TLC), the next step is to reduce the nitro to an amine by use of the sulfurated sodium borohydride.

The product is compound I wherein Y is a benzene radical, A is $NO_2$ and R is a 15'-digoxin radical.

Before proceeding, the coupled digoxin should be dried by rotary evaporation and washed once with tetrahydrofuran (THF) before placing the p-nitrophenylisocyanato-digoxin into the reaction vessel.

The reaction with $NaBH_2S_3$ can be carried out in dioxane or THF, THF being preferred because of

the solubility of the substituted digoxin in this solvent. Although it is not critical to maintain an atmosphere of nitrogen, the vessel should be purged, as described, prior to the addition of the $NaBH_2S_3$.

To a 100 ml round bottom, add the digoxin (substituted p-nitrophenylisocyanato-digoxin) and stir bar in a volume of THF. (Again, minimize volume of THF). Purge with nitrogen employing a vent needle, through a rubber septum for 5 minutes.

In a separate vessel, place the dried, dessicated, $NaBH_2S_3$, and add 20 mls of THF and dissolve completely, purge with nitrogen through a rubber septum.

With a 50 ml glass syringe, withdraw the solution and add slowly to the digoxin, with continuous stirring. The reaction rate can be followed by IR (conversion of $NO_2 \rightarrow NH_2$) and the reaction should be complete after only several hours, dependent on concentration of the digoxin. Heat can be employed to speed the forward rate, but only to 37°C, and should be monitored very closely. At 25°C (R.T.) the reaction proceeds somewhat more slowly, but more controllably.

This product can be isolated and purified by silica-gel TLC (preparative) or by column, silica-gel, and washed extensively with methanol, filtered and dried for storage; or, it can be used directly with no further purification, as the ensuing reactions will not involve the nitro in any way, and final subsequent purification could be used to purify these products.

Once the relative concentration of digoxin has been established (again by RIA) subsequent reactions with p-aminophenylisocyanate-digoxin, can proceed through use of, e.g., fluorescein isothiocyanate, directly.

Place 100 μ moles of substituted digoxin in a clear dry 25 ml round bottom, adding stir bar. Add, by syringe, 10 mls of pyridine, close with a rubber septum and purge with nitrogen, as described, stirring until the product is completely dissolved.

To a second round bottom, add 100 μ moles of fluorescein isothiocyanate and dissolve in the pyridine, 10—15 mls. Close with a rubber septum, purge with $N_2$, and remove by glass syringe and add slowly to the digoxin, using continuous mixing. (Due to the nature of fluorescein isothiocyanate, not all material will be transferred as it will stick to the round bottom and the syringe; however, if necessary, more can be added at a later point by the same method described.

This reaction will take about 72 hours to complete and can be monitored by the appearance of a new fluorescent specie if analytical TLC is employed).

Purification of the product must be done prior to further analytical workups, and analysis by RIA will demonstrate immunoreactivity of the final product, and any additional tests devised to detect fluorescence.

Once the final product is isolated, it should be extensively washed with methanol and filtered (0.22 μ) several times, dried by dessication and stored under dessication at 30°C (frozen).

The resulting product is a compound II wherein R is a 15′ digoxin radical;

$$Z \text{ is } -N-\overset{\overset{O}{\|}}{C}-N-B;$$

B is a fluorescein radical; and Y is a divalent benzene radical.

Example 3

To a 25 ml round bottom add 100 millimoles of 15′-p-aminophenylisocyanato digoxin (digoxin) (as prepared in Example 2) and add 5—7 mls of freshly distilled pyridine, dissolve and mix until completely dissolved by stirring. Cover the round bottom with a rubber septum and purge with $N_2$ for 5 minutes.

Remove by glass (clean and *very* dry) syringe 100 μ moles of this phosgene liquid, in a well ventilated fume hood, and expunge all air from the syringe and slowly add the thiophosgene to the continuously stirring digoxin solution, over 3—4 minutes. The reaction vessel should be kept out of direct light (foil or a box) and monitoring should be done by removing, by syringe, small aliquots and IR should indicate the formation of the isothiocyanate at $2250^{-cu}$; formation proceeds rapidly and is usually complete in several hours, but can be left overnight if desired.

The reaction is stopped by the addition of methanol and should be washed several times with methanol and dried by rotary evaporation.

Isolation and purification can be done by TLC, using methanol:chloroform (50:50) and removal of the para-isothiocyanate-phenylisocyanato-digoxin by extraction has been previously described (prep silica-gel, extraction in MeOH and filtering by 0.22 μ filters, dry, wash (3X) and dry for storage.

The resulting product is compound I wherein Y is a divalent benzene radical; R is 15′ digoxin radical and A is —N=C=S.

Once isolated and purified, this compound would be stable over several months before requiring any further purification. Storage conditions must be maintained. (They are dry, dessicated at −30°C).

Example 4

Reaction of 15′-p-isothiocyanatophenylisocyanato-digoxin with an enzyme (acid phosphatase).

Keeping reaction volumes to a minimum, to an open (or closed) vessel, place an appropriate amount of enzyme, dry, and add a minimum volume of a basic, low molar buffer which *does not* contain primary or secondary amines; e.g., borate buffer pH 9.5—0.5M would be appropriate. Stir by mixing at 4°C.

Calculate the degree of substitution (digoxin:enzyme) desired and to that amount add either dimethylformamide or dimethylsulfoxide equivalent to no more than 10% of the total volume of the enzyme solution. Mix until dissolved.

By pipette or similar transfer device, remove the digoxin in DMF or DMSO and add *very slowly* to the pH 9.5 solution containing the enzyme. (Should take 10 minutes and the receiving vessel should be kept cold, either stored at 4°C or on ice). The reaction to the formation of the digoxin-enzyme (complex) is relatively fast and is usually complete within 24 hours. The product is compound II wherein R is a digoxin radical; Y is a divalent benzene radical;

$$Z \text{ is } NH—\overset{\overset{S}{\|}}{C}—NH—B$$

and B is an acid phosphatase enzyme radical.

Purification of the conjugated digoxin-acid phosphatase is accomplished by employing any one of a number of separations such as sephadex, bio-gel, etc.

The above procedures are illustrative and may be similarly employed for coupling a wide variety of organic compounds to each other, provided that the organic compounds have the required active hydrogen substituted groups. Thus, the above procedures are applicable to the coupling of ligands other than digoxin or $T_4$ to organic compounds other than fluorescein dyes and/or enzymes.

The present invention is particularly advantageous in that by use of compound III it is possible to produce coupled products II in which there is a rigid coupling of one organic compound to another. This is of advantage when producing fluorescent tracers in that the rigidity of the coupling reduces quenching of the fluorescent compound by the ligand. Thus, for example, the heavy atom effect on a fluorescent material (for example, the iodine groups of $T_3$ and/or $T_4$), which can quench a fluorescent material is reduced and/or eliminated. Thus, the invention has particular use in the production of a thyroid hormone tracer wherein a thyroid hormone ($T_3$ or $T_4$) is coupled to a fluorescent compound.

## Claims

1. A compound of the general formula

R—CO—NH—Y—Z

wherein R is an organic radical derived from an organic compound RH in which the indicated H is an active hydrogen; Y is a divalent aromatic hydrocarbon radical; and Z is

—NH—CH—COB, —NH—CO—OB,
—NH—CO—SB, —NH—CO—NHB,
—NH—CS—NHB, —CO—NHB, —S—CO—NHB or
—O—CO—NHB,

wherein B is an organic radical, and wherein one of R and B comprises a detectable marker and the other comprises an antigen, hapten or antibody.

2. A compound according to claim 1, wherein Y is a divalent benzene radical.

3. A compound according to claim 1 or 2, wherein R is a hapten or an antigen.

4. A compound according to claim 3, wherein RH is digoxin.

5. A compound according to claim 3, wherein RH is $T_4$.

6. A compound according to claim 1, wherein R comprises the detectable marker.

7. A compound according to claim 1, wherein B comprises the detectable marker.

8. A compound according to any preceding claim, wherein the detectable marker is a fluorescent dye, preferably fluorescein.

9. An assay for an analyte by use of a tracer, wherein the tracer is a compound according to any preceding claim.

10. A compound of the general formula

R—CO—NH—Y—A

wherein R is an organic radical derived from an organic compound RH in which R is a detectable marker, an antibody, a hapten or an antigen and the indicated H is an active hydrogen, Y is as defined in claim 1 and A is $—NO_2$, $—NH_2$, —COOH, —COCl, —COOR'', —NCS, —SH, —OH, —NCO, —SCOR'' or —OCOR'' in which R'' is alkyl.

11. A compound according to claim 10, wherein A is $—NO_2$, $—NH_2$, —NCS or —NCO.

## Patentansprüche

1. Verbindung der allgemeinen Formel

R—CO—NH—Y—Z

worin R ein organischer, von einer organischen Verbindung RH, in der H ein aktives Wasserstoffatom ist, abgeleiteter Rest; Y ein bivalenter aromatischer Kohlenwasserstoffrest; und Z

—NH—CH—COB, —NH—CO—OB,
—NH—CO—SB, —NH—CO—NHB,
—NH—CS—NHB, —CO—NHB, —S—CO—NHB
oder —O—CO—NHB

ist, in welchen B ein organischer Rest ist, und worin einer der Reste R und B eine nachweisbare Markierungssubstanz und der andere ein Antigen, Hapten oder einen Antikörper umfaßt.

2. Verbindung nach Anspruch 1, worin Y ein bivalenter Benzolrest ist.

3. Verbindung nach Anspruch 1 oder 2, worin R ein Hapten oder ein Antigen ist.

4. Verbindung nach Anspruch 3, worin RH Digoxin ist.

5. Verbindung nach Anspruch 3, worin RH $T_4$ ist.

6. Verbindung nach Anspruch 1, worin R die nachweisbare Markierungssubstanz umfaßt.

7. Verbindung nach Anspruch 1, worin B die nachweisbare Markierungssubstanz umfaßt.

8. Verbindung nach einem der vorhergehenden Ansprüche, worin die nachweisbare Markierungssubstanz ein fluoreszierender Farbstoff, vorzugsweise Fluorescein, ist.

9. Bestimmung eines Analyts durch Verwendung eines Tracers, worin der Tracer eine Verbindung gemäß einem der vorhergehenden Ansprüche ist.

10. Verbindung der allgemeinen Formel

R—CO—NH—Y—A

worin R ein organischer, von einer organischen Verbindung RH, in der R eine nachweisbare Markierungssubstanz, eine Antikörper, Hapten oder Antigen und H ein aktives Wasserstoffatom ist, abgeleiteter Rest ist, Y die in Anspruch 1 genannte Bedeutung besitzt und A —$NO_2$, —$NH_2$, —COOH, —COCl, —COOR'', —NCS, —SH, —OH, —NCO, —SCOR'' oder —OCOR'', worin R'' Alkyl bedeutet, ist.

11. Verbindung nach Anspruch 10, worin A —$NO_2$, —$NH_2$, —NCS oder —NCO ist.

**Revendications**

1. Composé de formule générale

R—CO—NH—Y—Z

dans lequelle R est und radical organique dérivé d'un composé organique RH dans lequel le H indiqué est un hydrogène actif, Y est un radical hydrocarbure aromatique divalent; et Z est

—NH—CH—COB, —NH—CO—OB,
—NH—CO—SB, —NH—CO—NHB,
—NH—CS—NHB, —CO—NHB, —S—CO—NHB
ou —O—CO—NHB

dans lequel B est un radical organique, et dans lequel un de R et de B comprend un marqueur détectable et l'autre comprend un antigène, haptène ou anticorps.

2. Composé selon la revendication 1, dans lequel Y est un radical benzène divalent.

3. Composé selon la revendication 1 our 2, dans lequel R est un haptène ou un antigène.

4. Composé selon la revendication 3, dans lequel RH est la digoxine.

5. Composé selon la revendication 3, dans lequel RH est $T_4$.

6. Composé selon la revendication 1, dans lequel R comprend le marqueur détectable.

7. Composé selon la revendication 1, dans lequel B comprend le marqueur détectable.

8. Composé selon l'une quelconque des revendications précédentes, dans lequel le marqueur détectable est un colorant fluorescent, de préférence la fluorescéine.

9. Dosage d'un analyte par utilisation d'un traceur, dans lequel le traceur est un composé selon l'une quelconque des revendications précédentes.

10. Composé de formule générale

R—CO—NH—Y—A

dans lequel R est un radical organique dérivé d'un composé organique RH dans lequel R est un marqueur détectable, un anticorps, un haptène ou un antigène et le H indiqué est un hydrogène actif, Y est comme défini dans la revendication 1 et A est —$NO_2$, —$NH_2$, —COOH, —COCl, —COOR'', —NCS—, —SH, —OH, —NCO, —SCOR'' ou —OCOR'' dans lequel R'' est un radical alkyle.

11. Composé selon la revendication 10, dans lequel A est —$NO_2$, —$NH_2$, —NCS ou —NCO.